Europäisches Patentamt

European Patent Office

Office européen des brevets

(19)

(11) Numéro de publication: **0 136 927**
**A2**

## (12) DEMANDE DE BREVET EUROPEEN

(21) Numéro de dépôt: **84401511.5**

(22) Date de dépôt: **18.07.84**

(51) Int. Cl.⁴: **A 61 K 31/70**
**A 61 K 35/78**
**//(A61K31/70, 31:49),**
**(A61K35/78, 31:49)**

(30) Priorité: **04.08.83 FR 8312849**

(43) Date de publication de la demande:
**10.04.85 Bulletin 85/15**

(84) Etats contractants désignés:
**AT BE CH DE FR GB IT LI LU NL SE**

(71) Demandeur: **SYNTHELABO**
**58, rue de la Glacière**
**F-75621 Paris Cedex 13(FR)**

(72) Inventeur: **Balansard, Guy**
**12, rue du Camas**
**F-13005 Marseille(FR)**

(72) Inventeur: **Timon-David, Pierre**
**76, rue Saint-Savournin**
**F-13001 Marseille(FR)**

(74) Mandataire: **Thouret-Lemaitre, Elisabeth et al,**
**Service Brevets - SYNTHELABO 58, rue de la Glacière**
**F-75621 Paris Cedex 13(FR)**

(54) **Compositions pharmaceutiques contenant de la quinine.**

(57) Composition pharmaceutique caractérisée en ce qu'elle contient un alcaloïde ou un mélange d'alcaloïdes extrait de l'écorce de quinquina en association avec du tanin pur ou un extrait à 60% de tanins d'Annona senegalensis.

EP 0 136 927 A2

Croydon Printing Company Ltd

Compositions pharmaceutiques contenant de la quinine.

La présente invention concerne des compositions
pharmaceutiques à base de quinine.

La quinine est connue pour son activité antimalarique. La
Demanderesse a trouvé que des compositions pharmaceutiques
contenant une association de quinine et de tanin extrait
d'Annona senegalensis étaient particulièrement actives pour
le traitement de la malaria (ou paludisme).

Les compositions de l'invention contiennent de la quinine,
éventuellement sous forme de sel, en association avec le
tanin pur extrait d'Annona senegalensis ou un extrait à 60 %
de tanins, et éventuellement en association avec de la
cinchonine.

Les compositions pharmaceutiques de l'invention sont
beaucoup plus actives que la quinine elle-même.

La quinine et la cinchonine, extraites d'écorces de
quinquina, sont connues dans la littérature pour leur
activité antimalarique. Toutefois la cinchonine, légérement
plus toxique, ne peut être utilisée seule en thérapeutique.

Le tanin pur extrait d'Annona senegalensis et l'extrait à
60 % de tanins condensés sont obtenus à partir de l'écorce
de la tige de cette plante.

L'extrait renferme 60 % de tanins condensés accompagnés de
glucose, d'acides aminés et de peptides.

Le tanin pur a été isolé par chromatographie liquide
préparative en utilisant comme support de la silice greffée
RP8 (Merck) et comme éluant du méthanol contenant 70 %
d'eau, après élution successive par de l'eau distillée et de
l'eau contenant 10 % de méthanol.

Les dosages réalisés par la méthode utilisant le réactif de Stiasny montrent la seule présence de tanins condensés.

On prépare l'extrait purifié d'Annona senegalensis de la manière suivante :

On soumet l'écorce de la tige d'Annona senegalensis sèche et réduite en poudre à une lixiviation par 5 fois son poids d'un mélange méthanol 55/ eau 45 (en poids).

Le liquide obtenu est concentré sous pression réduite jusqu'à évaporation du méthanol.

La solution aqueuse résiduelle est extraite à deux reprises par un volume identique d'acétate d'éthyle.

Les fractions solubles dans l'acétate d'éthyle sont éliminées.

La phase aqueuse résiduelle est ensuite séchée par atomisation ou lyophilisation.

Le résidu est extrait à chaud et à deux reprises par dix fois son poids d'un mélange acétone/acétate d'éthyle (1/1 en volume).

L'insoluble est solubilisé dans l'eau et séché par atomisation ou lyophilisation.

L'extrait d'Annona senegalensis obtenu est une poudre marron clair, très soluble dans l'eau, partiellement soluble dans le méthanol et l'éthanol et insoluble dans l'acétone et l'éther.

Les tanins condensés contenus dans l'extrait ont été caractérisés par plusieurs tests et réactifs :

1. Le chauffage à l'ébullition de quelques mg de l'extrait dans 5 ml d'HCl 5N a conduit à la formation d'une coloration rouge intense. Après refroidissement, il y a précipitation d'un phlobaphène insoluble. Cette réaction est caractéristique des tanins condensés dérivés des flavane-diols.

2. Par addition de 50 ml du réactif de Stiasny (25 ml de formol à 40 % et 25 ml d'acide chlorhydrique) à une solution de 100 mg de l'extrait dans 100 ml d'eau, il se forme un précipité marron, caractéristique des tanins condensés.

3. L'addition de 0,5 ml d'une solution de $FeCl_3$ à 2 % à 5 ml d'une solution aqueuse à 0,5 % d'extrait conduit à une coloration verte, caractéristique de la présence de phénols.

4. L'addition à 2 ml d'une solution à 2 % d'extrait d'1 ml d'$H_2SO_4$ 0,1 N et de 3 ml d'une solution aqueuse de molybdate d'ammonium à 10 % conduit à une coloration jaune orangée caractéristique des orthodiphénols.

5. On dissout 0,2 g d'extrait dans 10 ml d'HCl 5N puis on porte la solution au bain marie pendant 2 minutes.

Après refroidissement, on extrait l'anthocyanidine formée par 1 ml d'alcool isoamylique. On identifie l'anthocyanidine par chromatographie.

|  | Extrait | Cyanidine témoin |
|---|---|---|
| Papier whatman n° 1 Solvant Forestal * | Rf 0,17 | 0,17 |
| CCM Cellulose Merck HCOOH-HCl-$H_2O$(10-1-3) | Rf 0,42 | 0,42 |
| CCM Cellulose Merck Alcool amylique- $CH_3COOH-H_2O$ (2-1-1) | Rf 0,46 | 0,46 |

Forestal * $CH_3COOH-HCl$ Conc.-$H_2O$ (5-3-10)

. Après chromatographie sur papier, les taches sont éluées par du méthanol Merck.

L'essai et le témoin présentent un maximum d'absorption à 537 nm en spectroscopie.

. Ces 2 essais permettent de caractériser la cyanidine après hydrolyse acide du tanin.

6. Après hydrolyse simple et oxydative de l'extrait en présence de $FeCl_3$, on sature la phase aqueuse avec du chlorure de sodium et on extrait par de l'éther.

La phase éthérée agitée avec une solution aqueuse de $FeCl_3$ à 0,5 % ne donne pas de coloration bleue caractéristique d'acide gallique, l'extrait ne contient donc pas de tanins hydrolysables de type gallique.

7. La recherche d'alcaloïdes et de saponosides est négative, contrairement à ce qui est décrit dans la littérature.

8. Spectrocopie dans l'ultra-violet :

. Le tanin pur en solution dans de l'eau distillée à la dilution de 25 µg/ml présente un maximum d'absorption à 279-280 nm. A 280 nm la DO doit être comprise entre 0,30 et 0,33.

L'extrait purifié à 60 % en solution dans de l'eau distillée à la dilution de 25 µg/ml présente un maximum d'absorption à 279-280 nm, la DO doit être comprise entre 0,18 et 0,20.

La caractérisation du glucose a été effectuée par réaction positive à la bandelette à la glucose-oxydase et par chromatographie en couche mince sur gel de silice GF 254 Merck.

Le solvant de développement est un mélange isopropanol/acide borique M/30 (85/15 en volume).

La révélation est effectuée par pulvérisation de phtalate d'aniline et chauffage à l'étuve à 105°C pendant 5 minutes.

L'essai et le témoin glucose présentent une tache de couleur marron, de Rf identique voisin de 0,5.

La caractérisation des acides aminés totaux est réalisée au moyen de l'amino-acide analyseur Carlo-Erba type 3A 27 B suivant la technique décrite par P. Molla, thèse de doctorat de spécialité, Marseille, 1975.

Après hydrolyse acide par HCl 5N ont été identifiés :

- 5 amino-acides basiques : acide gamma-aminobutyrique; ornithine, lysine, histidine, arginine.

12 amino-acides neutres : acide aspartique, thréonine, sérine, proline, acide glutamique, glycine, alanine, valine,

0136927

isoleucine, leucine,
tyrosine, phénylalanine.


Le dosage en tanins condensés de l'extrait a été effectué
par rapport au tanin pur isolé au moyen de la
chromatographie liquide préparative.

Les deux méthodes utilisées :

. méthode utilisant le réactif de Stiasny,
. méthode par spectroscopie en UV à 280 nm,
donnent un titre de 60 ± 3 %.


L'activité antimalarique des compositions de l'invention a
été étudiée in vivo sur des souris blanches infestées avec
le Plasmodium berghei.

Le Plasmodium berghei est le premier hématozoaire découvert
chez les rongeurs Muridés. Sa relative facilité d'entretien
et de multiplication chez les animaux courants de
laboratoire : souris et rat blancs, hamsters font de ce
parasite un matériel de choix pour la recherche des
nouvelles drogues antipaludiques.

Une substance a une activité antipaludique in vivo lorsqu'en
même temps : elle provoque une réduction de la parasitémie
et une prolongation de la survie des souris traitées.

Si la plupart des auteurs utilisent le test en quatre jours
(4 days test) qui permet de rechercher les DE 50 et DE 90 au
bout de 4 jours de traitement des souris parasitées, nous
suivrons l'évolution de la parasitémie des souris traitées
jusqu'à leur mort ou guérison éventuelle. Cette méthodologie
qui a été utilisée par Peters pour l'étude de la résistance
du Plasmodium berghei à la chloroquine, permet d'établir une
relation étroite entre l'évolution de la parasitémie et la
survie des souris traitées.

Les souris blanches pesant 20 ± 2 g sont infestées par voie intrapéritonéale avec 0,05 ml de sang contenant environ 2.10$^7$ globules rouges parasités.

Chaque lot comprend 10 souris et un lot témoin est constitué pour chaque série de test.

Les produits sont mis en suspension dans de l'eau distillée stérile. L'administration se fait par voie orale ; le traitement des souris commence le jour de l'infestation et se poursuit pendant 5 jours par l'administration d'une dose quotidienne à chaque souris. L'examen des frottis se fait à partir du troisième ou quatrième jour. On détermine en fin d'expérience le pourcentage d'hématies parasitées.

Dans un premier temps on a déterminé la dose minimale de chlorhydrate de quinine capable de réduire la parasitémie et de provoquer une prolongation de la survie des souris traitées par rapport aux témoins.

Cette dose est d'environ 80 mg/kg. A cette dose, le chlorhydrate de quinine provoque une diminution de la parasitémie pendant la durée du traitement. Les souris traitées survivent quelque temps par rapport aux souris témoins.

Aux doses de 120 et 150 mg/kg de chlorhydrate de quinine, les pourcentages de parasitémie des souris traitées diminuent. Les souris survivantes ne présentent pas de parasites après 30 jours de contrôle.

Le chlorhydrate de cinchonine, à la dose de 120 mg/kg, a la même activité que le chlorhydrate de quinine à la même dose.

A titre d'exemples, deux compositions de l'invention, contenant du chlorhydrate de quinine et du tanin pur (100 mg/kg) ont été étudiées. Les produits sont administrés à raison de 120 mg/kg et 150 mg/kg de chlorhydrate de quinine et de 100 mg/kg de tanin.

Les taux de mortalité des souris diminuent très fortement lorsque les souris sont traitées avec les compositions de l'invention.

Le tableau suivant (I) donne les résultats comparatifs obtenus avec les compositions de l'invention et avec le chlorhydrate de quinine seul.

TABLEAU I

| Délai de contrôle | Pourcentage de parasitémie des souris traitées (groupes de 10 souris) | | | |
|---|---|---|---|---|
| | Chlorhydrate de quinine (120 mg/kg) | Chlorhydrate de quinine (120 mg/kg) et tanin (100 mg/kg) | Chlorhydrate de quinine (150 mg/kg) | Chlorhydrate de quinine (150 mg/kg) et tanin (100 mg/kg) |
| To + 3 jours | 0,5 % | 0,8 % | 0,1 % | 0 % |
| To + 7 jours | 0 % | 0 % | 0 % | 0 % |
| To + 9 jours | 0,3 % | 0 % | 0,1 % | 0 % |
| To + 10 jours | 1,6 % | 1 % | 0,4 % | 0 % |
| To + 11 jours | 9 % | 1,5 % | 2 % | 0 % |
| To + 12 jours | 28 % | 3,5 % | 5,8 % | 0,2 % |
| To + 14 jours | 34 % | 8 % | 16,5 % | 0,3 % |
| To + 15 jours | 52 % | 16,5 % | 24 % | 1,5 % |
| To + 16 jours | 62 % | 18 % | 25 % | 3,5 % |
| To + 17 jours | | 18,5 % | 19 % | 7,5 % |
| To + 18 jours | | 19 % | 16 % | 11 % |
| To + 21 jours | | 21 % | 0 % | 12 % |
| To + 25 jours | | 12 % | 0 % | 8 % |
| To + 26 jours | | 0 % | 0 % | 0 % |
| To + 28 jours | | 0 % | 0 % | 0 % |
| To + 30 jours | | 0 % | 0 % | 0 % |
| Pourcentage d'animaux guéris | 0 % | 30 % | 30 % | 50 % |

A titre d'exemple une composition de l'invention, contenant du chlorhydrate de quinine (administré à raison de 80 mg/kg), du chlorhydrate de cinchonine (administré à raison de 40 mg/kg) et du tanin pur (administré a raison de 100 mg/kg) a été étudiée comparativement au mélange des chlorhydrates de quinine et de cinchonine.

Les résultats sont donnés dans le tableau suivant (II).

TABLEAU II

| Délai de contrôle | Pourcentage de parasitemie des souris traitées (groupe de 10 souris) | |
| --- | --- | --- |
| | Chl. quinine:80 mg/kg<br>Chl. cinchonine:40mg/kg | Chl. quinine:80 mg/kg<br>Chl. cinchonine:40mg/kg<br>Tanin : 100 mg/kg |
| To + 3 jours | 0,4 % | 0,2 % |
| To + 4 jours | 0,1 % | 0,1 % |
| To + 5 jours | 0 % | 0 % |
| To + 7 jours | 0 % | 0 % |
| To + 8 jours | 0 % | 0 % |
| To + 9 jours | 0 % | 0 % |
| To + 10 jours | 0 % | 0 % |
| To + 11 jours | 0,1 % | 0 % |
| To + 12 jours | 0,2 % | 0,1 % |
| To + 13 jours | 1 % | 0,1 % |
| To + 14 jours | 6 % | 0 % |
| To + 15 jours | 11 % | 0 % |
| To + 17 jours | 12 % | 0 % |
| To + 18 jours | 16 % | 0 % |
| To + 19 jours | 20 % | 0 % |
| To + 21 jours | 26 % | 0 % |
| To + 23 jours | 18 % | 0 % |
| To + 24 jours | 0 % | 0 % |
| To + 25 jours | 0 % | 0 % |
| To + 27 jours | 0 % | 0 % |
| To + 29 jours | 0 % | 0 % |
| To + 30 jours | 0 % | 0 % |
| Pourcentage d'animaux guéris | 30% | 100 % |

Il ressort de ces résultats que le tanin d'Annona senegalensis, lui-même non actif, potentialise l'activité de la quinine et l'activité de la quinine et de la cinchonine.

La synergie observée permet d'utiliser de moins grandes quantités de quinine pour le traitement ou la prévention de la malaria.

Les compositions de l'invention peuvent être préparées de telle manière que soit administrée chez l'homme

1°) pour un traitement curatif : une quantité de sel de quinine correspondant à 6 à 15 mg/kg de quinine base ou d'un mélange contenant 2/3 de sel de quinine et 1/3 de sel de cinchonine soit de 4 à 10 mg/kg de quinine et de 2 à 5 mg/kg de cinchonine et de 5 à 15 mg/kg de tanin pur d'Annona senegalensis ou une quantité de 8 à 25 mg/kg d'extrait d'Annona senegalensis titré à 60% de tanin.

2°) pour un traitement préventif une quantité de sel de quinine correspondant à 1 à 2,5 mg/kg/jour de quinine base ou d'un mélange contenant 2/3 de sel de quinine et 1/3 de sel de cinchonine, soit 0,66 à 1,6 mg/kg de quinine et 0,33 à 0,8 mg/kg de cinchonine et de 1 à 2,5 mg/kg de tanin pur d'Annona senegalensis ou une quantité de 1,5 à 4,5 mg/kg d'extrait d'Annona senegalensis titré à 60% de tanin.

Les toxicités de la quinine et de la cinchonine, testées sous forme de chlorhydrates chez la souris de 20 g $\pm$ 2 g sont les suivantes :
Les DL 50 sont proches et de l'ordre de 820 mg/kg pour le chlorhydrate de quinine et de 720 mg/kg pour le chlorhydrate de cinchonine.

Les toxicités de l'extrait d'Annona senegalensis et du tanin pur sont les suivantes : la DL 50 de l'extrait à 60 % est supérieure à 4 g/kg par voie orale, la DL 50 du tanin pur est d'environ 2,5 g/kg par voie orale.

Les compositions de l'invention peuvent contenir de 45 à 65 % en poids de quinine ou du mélange quinine-cinchonine et de 35 % à 55 % en poids de tanin pur.

Les compositions de l'invention peuvent être utilisées pour le traitement ou la prévention du paludisme.

Elles peuvent être présentées en association avec tout excipient approprié pour l'administration par voie orale ou parentérale.

**0136927**

Revendications
(pour tous états contractants : F, RFA, GB, B, I, LU, NL, SE, CH et
LI ; sauf Autriche)

1. Composition pharmaceutique caractérisée en ce qu'elle con
tient un alcaloïde ou un mélange d'alcaloïdes extrait de
l'écorce de quinquina en association avec du tanin pur ou
un extrait à 60 % de tanins d'Annona senegalensis.

2. Composition pharmaceutique selon la revendication 1,
caractérisée en ce que l'alcaloïde est la quinine,
éventuellement sous forme de sel.

3. Composition pharmaceutique selon la revendication 1,
caractérisée en ce qu'elle contient un mélange de quinine
et de cinchonine, éventuellement sous forme de sels.

4. Composition pharmaceutique selon la revendication 1,
caractérisée en ce qu'elle contient du chlorhydrate de
quinine.

5. Composition pharmaceutique selon la revendication 1,
caractérisée en ce qu'elle contient un mélange constitué
par 2/3 de sel de quinine et 1/3 de sel de cinchonine.

6. Composition pharmaceutique selon la revendication 1,
caractérisée en ce qu'elle contient de 45 % à 65 % en
poids d'alcaloïde base et de 35 % à 55 % en poids de
tanin pur.

7. Composition pharmaceutique selon la revendication 1,
caractérisée en ce qu'elle est préparée de manière à ce
que soient administrés de 6 à 15 mg/kg de quinine base et
de 5 à 15 mg/kg de tanin pur d'Annona senegalensis ou de
8 à 25 mg/kg d'extrait à 60% de tanins d'Annona
senegalensis.

8. Composition pharmaceutique selon la revendication 1, caractérisée en ce qu'elle est préparée de manière à ce que soient administrés de 4 à 10 mg/kg de quinine base et de 2 à 5 mg/kg de cinchonine base et de 5 à 15 mg/kg de tanin pur d'Annona senegalensis ou de 8 à 25 mg/kg d'extrait d'Annona senegalensis titré à 60% de tanins.

9. Composition pharmaceutique selon la revendication 1, caractérisée en ce qu'elle est préparée de manière à ce que soient administrés de 1 à 2,5 mg/kg de quinine base et de 1 à 2,5 mg/kg de tanin pur d'Annona senegalensis ou de 1,5 à 4,5 mg/kg d'extrait à 60% de tanins d'Annona senegalensis.

10. Composition pharmaceutique selon la revendication 1, caractérisée en ce qu'elle est préparée de manière à ce que soient administrés de 0,66 à 1,6 mg/kg de quinine base et de 0,33 à 0,8 mg/kg de cinchonine base et de 1 à 2,5 mg/kg de tanin pur d'Annona senegalensis ou de 1,5 à 4,5 mg/kg d'extrait d'Annona senegalensis titré à 60% de tanins.

Revendications (pour l'Autriche) 1. Procédé de préparation de compositions pharmaceutiques contenant un alcaloïde ou un mélange d'alcaloïdes extrait de l'écorce de quinquina en association avec du tanin pur ou un extrait à 60% de tanins d'Annona senegalensis, procédé caractérisé en ce que l'on mélange l'alcaloïde ou le mélange d'alcaloïdes et le tanin pur ou l'extrait à 60% de tanins.

2. Procédé selon la revendication 1, caractérisé en ce que l'alcaloïde est la quinine, éventuellement sous forme de sel.

3. Procédé selon la revendication 1, caractérisé en ce que le mélange d'alcaloïdes est un mélange de quinine et de cinchonine, éventuellement sous forme de sels.

4. Procédé selon la revendication 1, caractérisé en ce que l'alcaloïde est le chlorhydrate de quinine.

5. Procédé selon la revendication 1, caractérisé en ce que le mélange d'alcaloïdes est constitué par 2/3 de sel de quinine et 1/3 de sel de cinchonine.

6. Procédé selon la revendication 1, caractérisé en ce que l'on mélange de 45% à 65% en poids d'alcaloïde base et de 35% à 55% en poids de tanin pur.